# NEW EUROPEAN PATENT SPECIFICATION

(11) **EP 3 860 768 B2**
(45) Date of publication and mention of the opposition decision: **15.07.2026**
(45) Mention of the grant of the patent: 22.05.2024
(21) Application number: 19868411.0
(22) Date of filing: 01.10.2019
(51) Int. Cl.: B05B 17/06, A61M 15/00

(54) **DELIVERY OF LOW SURFACE TENSION COMPOSITIONS TO THE PULMONARY SYSTEM VIA ELECTRONIC BREATH ACTUATED DROPLET DELIVERY DEVICE**
ABGABE VON ZUSAMMENSETZUNGEN MIT NIEDRIGER OBERFLÄCHENSPANNUNG AN DAS PULMONALE SYSTEM ÜBER EINE ELEKTRONISCHE ATEMZUGBETRIEBENE TRÖPFCHENABGABEVORRICHTUNG
ADMINISTRATION DE COMPOSITIONS À FAIBLE TENSION DE SURFACE AU SYSTÈME PULMONAIRE PAR L'INTERMÉDIAIRE D'UN DISPOSITIF ÉLECTRONIQUE DE DISTRIBUTION DE GOUTTELETTES ACTIONNÉ PAR LA RESPIRATION

(30) Priority: 01.10.2018 US 201862739740 P
(43) Date of publication of application: 11.08.2021
(60) Divisional application: 24176874.6
(73) Proprietor: Pneuma Respiratory, Inc., Boone, NC 28607 (US)
(72) Inventor: HUNTER, Charles Eric, Boone, NC 28607 (US); THOMAS, Brian H., Boone, NC 28607 (US); HEBRANK, John H., Boone, NC 28607 (US); CLEMENTS, Judson Sidney, Boone, NC 28607 (US)
(74) Representative: Greaves Brewster LLP
(86) International application number: PCT/US2019/054042
(87) International publication number: WO 2020/072478

(56) References cited:
- WO-A1-2015/036764
- WO-A1-2016/164917
- WO-A1-2017/149165
- US-A- 6 062 212
- US-A1- 2004 195 403
- US-A1- 2005 236 501
- US-A1- 2005 236 501
- US-A1- 2013 150 812
- US-A1- 2017 319 796
- US-A1- 2017 319 796
- THIAGO C. CARVALHO ET AL: "The function and performance of aqueous aerosol devices for inhalation therapy", JOURNAL OF PHARMACY AND PHARMACOLOGY : JPP, vol. 68, no. 5, 8 April 2016 (2016-04-08), GB, pages 556 - 578, XP055730729, ISSN: 0022-3573, DOI: 10.1111/jphp.12541

## Description

### FIELD OF THE INVENTION

This disclosure relates to the delivery of agents to the pulmonary via an inhalation delivery device, and more specifically via an electronically actuated droplet delivery device for delivering a low surface tension composition as an ejected stream of droplets to the pulmonary system of a subject.

### BACKGROUND OF THE INVENTION

The use of aerosol generating devices for the delivery of substances to the pulmonary system is an area of large interest. A major challenge is providing a device that delivers an accurate, consistent, and verifiable dose, with a droplet size that is suitable for successful delivery of substances to the targeted passageways.

Aerosol verification, delivery and inhalation of the correct amount at the desired times is important. A need exists to insure that users correctly use aerosol devices, and that they administer the proper amount at desired time. Problems emerge when users misuse or incorrectly delivery substances to the pulmonary system,

An electronically actuated droplet delivery device is disclosed in document US2017/319796.

Droplets with diameters between 0.5 µm and 7 µm will effectively deposit substances in the lung. Droplets larger than this range of sizes are mostly deposited in the mouth and upper respiratory tract, and droplets smaller than this range mostly fail to settle and are exhaled. Compositions intended for quick systemic uptake via pulmonary delivery typically target the alveolar region where the blood-gas interface provides rapid transport of substances from the alveoli to the bloodstream.

There is a need for improved methods and devices for delivering droplets to the pulmonary systems via a droplet delivery device.

### SUMMARY OF THE INVENTION

The invention is defined by the appended claims. Subject-matter referred as embodiments and/or disclosures which are not claimed are not part of the invention.

Certain aspects of the disclosure relate to an electronically actuated droplet delivery device for delivering a low surface tension composition as an ejected stream of droplets to the pulmonary system of a subject. In some embodiments, the low surface tension composition comprises an alcohol as a solvent. In other embodiments, the low surface tension composition is a composition comprising an agent that is insoluble or sparingly solution in water.

In one embodiment, the device comprises a housing; a mouthpiece positioned at an airflow exit of the device; a reservoir disposed within or in fluid communication with the housing containing a low surface tension composition; an electronically actuated ejector mechanism in fluid communication with the reservoir and configured to generate the ejected stream of droplets; and at least one differential pressure sensor positioned within the housing, the at least one differential pressure sensor configured to activate the ejector mechanism upon sensing a pre-determined pressure change within the mouthpiece to thereby generate the ejected stream of droplets.

In certain embodiments, the ejector mechanism comprises a piezoelectric actuator and an aperture plate, the aperture plate having and a plurality of openings formed through its thickness and one or more surfaces configured to provide a surface contact angle of greater than 90 degrees, and the piezoelectric actuator operable to oscillate the aperture plate at a frequency to thereby generate the ejected stream of droplets.

In some embodiments, the ejector mechanism is configured to generate the ejected stream of droplets wherein at least about 50% of the droplets have an average ejected droplet diameter of less than about 6 microns, such that at least about 50% of the mass of the ejected stream of droplets is delivered in a respirable range to the pulmonary system of the subject during use.

In some embodiments, the aperture plate has one or more surfaces configured to provide a surface contact angle of between 90 degrees and 140 degrees.

In yet other embodiments, the aperture plate is coated with a hydrophobic polymer to provide said surface contact angle. In some embodiments, the hydrophobic polymer is selected from the group consisting of polytetrafluoroethylene, a siloxane, paraffin, and polyisobutylene. In yet other embodiments, the hydrophobic polymer coating is chemically or structurally modified or treated.

In some embodiments, the hydrophobic polymer is coated on at least a portion of droplet exit side surface of the aperture plate. In other embodiments, the hydrophobic polymer is coated within at least a portion of the interior of at least one of the openings.

In some aspects, the droplet delivery device further includes an air inlet flow element positioned in the airflow at the airflow entrance of the device and configured to facilitate non-turbulent (i.e., laminar and/or transitional) airflow across the exit side of aperture plate and to provide sufficient airflow to ensure that the ejected stream of droplets flows through the droplet delivery device during use. In some embodiments, the air inlet flow element may be positioned within the mouthpiece.

In certain embodiments, the housing and ejector mechanism are oriented such that the exit side of the aperture plate is perpendicular to the direction of airflow and the stream of droplets is ejected in parallel to the direction of airflow. In other embodiments, the housing and ejector mechanism are oriented such that the exit side of the aperture plate is parallel to the direction of airflow and the stream of droplets is ejected substantially perpendicularly to the direction of airflow such that the ejected stream of droplets is directed through the housing at an approximate 90 degree change of trajectory prior to expulsion from the housing.

In certain aspects, the droplet delivery device further includes a surface tension plate between the aperture plate and the reservoir, wherein the surface tension plate is configured to increase contact between the volume of fluid and the aperture plate. In other aspects, the ejector mechanism and the surface tension plate are configured in parallel orientation. In yet other aspects, the surface tension plate is located within 2 mm of the aperture plate so as to create sufficient hydrostatic force to provide capillary flow between the surface tension plate and the aperture plate.

In yet other aspects, the aperture plate of the droplet delivery device comprises a domed shape. In other aspects, the aperture plate may be formed of a metal, e.g., stainless steel, nickel, cobalt, titanium, iridium, platinum, or palladium or alloys thereof. Alternatively, the plate can be formed of suitable material, including other metals or polymers, In other aspects.

In certain embodiments, the aperture plate is comprised of, e.g., poly ether ether ketone (PEEK), polyimide, polyetherimide, polyvinylidine fluoride (PVDF), ultra-high molecular weight polyethylene (UHMWPE), nickel, nickel-cobalt, palladium, nickel-palladium, platinum, or other suitable metal alloys, and combinations thereof. In other aspects, one or more of the plurality of openings of the aperture plate have different cross-sectional shapes or diameters to thereby provide ejected droplets having different average ejected droplet diameters.

In yet other aspects, the reservoir of the droplet delivery device is removably coupled with the housing. In other aspects, the reservoir of the droplet delivery device is coupled to the ejector mechanism to form a combination reservoir/ejector mechanism module, and the combination reservoir/ejector mechanism module is removably coupled with the housing.

In other aspects, the droplet delivery device may further include a wireless communication module. In some aspects, the wireless communication module is a Bluetooth transmitter.

In yet other aspects, the droplet delivery device may further include one or more sensors selected from an infer-red transmitter, a photodetector, an additional pressure sensor, and combinations thereof.

In another aspect, the disclosure relates to a method (not being part of the invention) for delivering a low surface tension composition as an ejected stream of droplets in a respirable range to the pulmonary system of a subject, the method comprising: (a) generating an ejected stream of droplets from the low surface tension composition via a an electronically actuated droplet delivery device of the disclosure, wherein at least about 50% of the ejected stream of droplets have an average ejected droplet diameter of less than about 6 µm; and (b) delivering the ejected stream of droplets to the pulmonary system of the subject such that at least about 50% of the mass of the ejected stream of droplets is delivered in a respirable range to the pulmonary system of a subject during use.

In some embodiments, the low surface tension composition comprises an alcohol as a solvent. In other embodiments, the low surface tension composition comprises an agent that is insoluble or sparingly soluble in water. In some embodiments, agent that is insoluble or sparingly soluble in water is selected from the group consisting of cannabinoids and derivatives thereof, fluticasone furoate, and fluticasone propionate.

In other embodiments, the low surface tension composition is delivered to a subject to treat or ameliorate a disease, condition or disorder selected from the group consisting of asthma, COPD epilepsy, seizure disorders, pain, chronic pain, neuropathic pain, headache, migraine, arthritis, multiple sclerosis, anorexia, nausea, vomiting, anorexia, loss of appetite, anxiety, or insomnia.

While multiple embodiments are disclosed, still other embodiments of the present disclosure will become apparent to those skilled in the art from the following detailed description, which shows and describes illustrative embodiments of the disclosure. Accordingly, the detailed descriptions are to be regarded as illustrative in nature and not restrictive.

### BRIEF DESCRIPTION OF THE DRAWINGS

**FIGS. 1A-1B** illustrate perspective views of an exemplary droplet delivery device, in accordance with embodiments of the disclosure.
**FIG. 2** is an exploded view of droplet delivery device of **FIG. 1A-1B****,** in accordance with embodiments of the disclosure.
**FIG. 3A** is a partial cross section perspective view of an in-line droplet delivery device of **FIG. 1A-1B** comprising a drug delivery ampoule, mouthpiece including an air inlet flow element, and mouthpiece cover, in accordance with an embodiment of the disclosure.
**FIG. 3B** is a front view of an in-line droplet delivery device of **FIG. 1A-1B** comprising a drug delivery ampoule and mouthpiece including an air inlet flow element, in accordance with an embodiment of the disclosure.
**FIG. 3C** is a exploded view of components of an in-line droplet delivery device of **FIG. 1A-1B** including a mouthpiece and internal housing, in accordance with an embodiment of the disclosure.
**FIGS. 4A-4B** illustrate perspective views of another exemplary droplet delivery device, in accordance with embodiments of the disclosure.
**FIG. 5** is a perspective view of a droplet delivery device of **FIG. 4A-4B** without the fluid reservoir/ejector mechanism module inserted, in accordance with embodiments of the disclosure.
**FIGS. 6A-6B** are perspective views of a fluid reservoir/ejector mechanism module and mouthpiece cover, showing a front view (**FIG. 6A**) and back view (**FIG.6B**), in accordance with embodiments of the disclosure.
**FIG. 7** illustrates a cross-section of an exemplary opening configured to provide a desired surface tension, in accordance with an embodiment of the disclosure.

### DETAILED DESCRIPTION

Effective delivery of low surface tension compositions, particularly those that include agents that are insoluble or sparingly soluble in water via an aerosol generating inhalation device has always posed a problem. Given their lack of water solubility, such agents are often formulated in low surface tension compositions, such as non-aqueous solutions, emulsions, suspensions, or encapsulations.

In accordance with aspects of the disclosure, any known methodology of forming compositions of non-water soluble/sparingly soluble agents may be used to prepare such formulations, including a non-aqueous solutions, compositions using alcohol as a solvent, formation of suspensions, formation of emulsions, lipid encapsulation, etc. Embodiments of the disclosure includes device and methods for delivering low surface tension compositions, particularly compositions of agents that are insoluble or sparingly soluble in water, e.g., as non-aqueous solutions, solutions with alcohol as a solvent, lipid encapsulations emulsions (e.g., oil/water), suspensions, etc.

The agent that is insoluble or sparingly soluble in water is any known agent that has no or low solubility in water, e.g., less than about 500 mg/L at 25 °C.

By way of non-limiting example, agents that are insoluble or sparingly soluble in water may generally be soluble in alcohols, including ethanol and isopropanol. As such, in certain aspects of the disclosure, solutions of these agents may be prepared by dissolving the agent or extracting the agent into the alcohol solvent. For example, in certain embodiments, cannabinoids may be extracted from cannabis plant into alcohol solvent solutions, which may optionally include various excipients. In certain aspects, the alcohol solutions may have low water content, e.g., at least 50% alcohol, 75%, 90%, 95%, 100% alcohol. In particular embodiments, 95% USP or 100% ethanol may be used to dissolve or extract the agent.

In other embodiments, encapsulations or emulsions may be formed from the agent using any known, e.g., lipid encapsulating or emulsion technology, e.g., cyclodextrin, phospholipids (e.g., phosphatidylcholine), oil/water, etc. Once the agent is lipid encapsulated or an emulsion is formed, any known technique may be used to form nano-lipids or a nano-emulsion, e.g., ultra-sonication, milling, etc., such that the nano-lipids or nano-emulsions are less than 5 µm, less than 4 µm, less than 3 µm, etc. In this regard, not only may the nano-lipids or nano-emulsions be sized so that the generated droplets are able to penetrate into the pulmonary system (as described herein), but the nano-lipids may also be sized such that they do not block the openings of the ejector mechanism of the droplet delivery device (described in further detail herein).

In this regard, certain aspects of the disclosure relate to an electronic breath actuated aerosol delivery devices that are particularly configured for the delivery of low surface tension compositions, particularly low surface tension compositions including agents that insoluble or sparingly soluble in water to the pulmonary system, described herein as a droplet delivery device or soft mist inhaler (SMI) device. In other aspects, the disclosure provides methods for delivery of low surface tension compositions to the pulmonary system via an electronic breath actuated aerosol delivery devices that are particularly configured for the delivery such compositions.

In certain embodiments, the present disclosure provides a droplet delivery device for delivery of a fluid as an ejected stream of droplets to the pulmonary system of a subject, the device comprising a housing, a reservoir for receiving a low surface tension composition, and an ejector mechanism including a piezoelectric actuator and an aperture plate, wherein the ejector mechanism is configured to eject a stream of droplets having an average ejected droplet diameter of less than about 5-6 microns, preferably less than about 5 microns. As discussed herein, in certain aspects, the droplet delivery device may include an ejector mechanism having an aperture plate having at least one surface of the aperture plate configured to facilitate generation of droplets from a low surface tension composition.

In certain embodiments, to facilitate generation of droplets from a low surface tension composition, one or more surfaces of the aperture plate may be configured (e.g., treated, coated, surface modified, or a combination thereof) to provide a desired surface contact angle of greater than 80 degrees, e.g., greater than 90 degrees, between 90 degrees and 140 degrees, between 90 degrees and 135 degrees, between 100 degrees and 140 degrees, between 100 degrees and 135 degrees, between 90 degrees and 110 degrees, etc. In various embodiments of the disclosure, the aperture plate may be configured to provide the desired surface contact angle at one or more surfaces, e.g., at least at a portion of the droplet exit surface, within at least one opening, at least at a portion of the droplet entrance surface, and combinations thereof. In particular embodiments, the aperture plate may provide the desired surface contact angle at least at a portion of the droplet exit surface or at least at a portion of the droplet exit surface and within at least one opening.

In certain embodiments, the aperture plate may be configured (e.g., treated, coated, surface modified, or a combination thereof) on one or more surfaces with a hydrophobic polymer to achieve the desired surface contact angle. In particular embodiments, the droplet exit side surface of the aperture plate is configured (e.g., treated, coated, surface modified, or a combination thereof) with a hydrophobic polymer. Any known hydrophobic polymer suitable for use in medical applications may be used, e.g., polytetrafluoroethylene (Teflon), a siloxane, paraffin, polyisobutylene, etc. The surface of the hydrophobic coating may be chemically or structurally modified or treated to further enhance or control the surface contact angle, as desired.

In accordance with aspects of the disclosure, exemplary methods for creating a hydrophobic surface on the aperture plate include dip coating methods and chemical deposition methods. Dip coating methods comprise dipping the aperture plate into a solution comprising a desired coating and a solvent, which solution will form a coating on the aperture plate when the solvent evaporates. Chemical depositions methods include known deposition methods, e.g., plasma etch, plasma coating, plasma deposition, CVD, electroless plating, electroplating, etc., wherein the chemical deposition uses a plasma or vapor to open the bonds on the surface of the aperture plate so that oxygen or hydroxyl molecules attach to the surface rendering it polar. In certain embodiments, the deposited hydrophobic layer is significantly thinner than the opening size such that it does not impact the size of the generated droplets.

In accordance with aspects of the disclosure, compositions comprising an agent that is insoluble or sparingly soluble in water may have a low surface tension (i.e., a surface tension lower than that of water). For instance, alcohols generally have a surface tension lower than water. By way of example, ethanol has a surface energy of 20 milliNewtons per meter, which is lower than water (about 73 mN/M). A 5% ethanol in water solution has a surface energy of 42 mN per meter. Further, lipid bilayers and emulsions may often exhibit lower surface energies than water. Without being limited by theory, in certain aspects of the disclosure, the ejector mechanism of an exemplary droplet delivery device of the disclosure is able to more effectively generate droplets of lower surface tension solutions, e.g., ethanol solutions, if the aperture plate surface exhibits a contact angle more compatible with the solution to be ejected.

In certain embodiments, the agent that is insoluble or sparingly soluble in water may isolated or derived from cannabis. For instance, the agent may be a natural or synthetic cannabinoid, e.g., THC (tetrahydrocannabinol), THCA (tetrahydrocannabinolic acid), CBD (cannabidiol), CBDA (cannabidiolic acid), CBN (cannabinol), CBG (cannabigerol), CBC (cannabichromene), CBL (cannabicyclol), CBV (cannabivarin), THCV (tetrahydrocannabivarin), CBDV (cannabidivarin), CBCV (cannabichromevarin), CBGV (cannabigerovarin), CBGM (cannabigerol monomethyl ether), CBE (cannabielsoin), CBT (cannabicitran), and various combinations thereof. In other embodiments, the agent may be a ligand that bind the cannabinoid receptor type 1 (CB₁), the cannabinoid receptor type 2 (CB₂), or combinations thereof. In particular embodiments, the agent may comprise THC, CBD, or combinations thereof. By way of example, the agent may comprise 95% THC, 98% THC, 99% THC, 95% CBD, 98% CBD, 99% CBD, etc.

In other embodiments, the agent that is insoluble or sparingly soluble in water may be fluticasone furoate, fluticasone propionate, and other generally water insoluble asthma and chronic obstructive pulmonary disease (COPD) medications.

In some embodiments, the composition comprising an agent that is insoluble or sparingly solution in water is a composition comprising alcohol as a solvent. In other embodiments, the agent is selected from the group consisting of cannabinoids and derivatives thereof, fluticasone furoate, and fluticasone propionate.

In certain embodiments, the methods and droplet delivery devices of the disclosure may be used to treat various diseases, disorders and conditions by delivering agents to the pulmonary system of a subject. In this regard, the droplet delivery devices may be used to deliver agents both locally to the pulmonary system, and systemically to the body. In certain embodiments, the methods and droplet delivery devices of the disclosure may be used to treat epilepsy, seizure disorders, pain, chronic pain, neuropathic pain, headache, migraine, arthritis, multiple sclerosis, anorexia, nausea, vomiting, anorexia, loss of appetite, anxiety, insomnia, etc. In other embodiments, the methods and droplet delivery devices of the disclosure may be used to treat asthma and/or COPD.

In other embodiments, the composition is delivered to a subject to treat or ameliorate a disease, condition or disorder selected from the group consisting of asthma, COPD epilepsy, seizure disorders, pain, chronic pain, neuropathic pain, headache, migraine, arthritis, multiple sclerosis, anorexia, nausea, vomiting, anorexia, loss of appetite, anxiety, or insomnia.

In certain embodiments, the droplet delivery device of the disclosure may be used to deliver scheduled and controlled substances such as cannabinoids. In certain embodiments, by way of non-limiting example, dosing may only enabled by user, doctor or pharmacy communication to the device, only in a specific location such as the user's residence as verified by GPS location on the user's smart phone, and/or it may be controlled by monitoring compliance with dosing schedules, amounts, abuse compliances, etc. In certain aspects, this mechanism of highly controlled dispensing of controlled agents can prevent the abuse or overdose of controlled substances.

In specific embodiments, the ejector mechanism is electronically breath activated by at least one differential pressure sensor located within the housing of the droplet delivery device upon sensing a pre-determined pressure change within the housing. In certain embodiments, such a pre-determined pressure change may be sensed during an inspiration cycle by a user of the device, as will be explained in further detail herein.

In accordance with certain aspects of the disclosure, effective deposition into the lungs generally requires droplets less than about 5-6 µm in diameter. Without intending to be limited by theory, to deliver fluid to the lungs a droplet delivery device must impart a momentum that is sufficiently high to permit ejection out of the device, but sufficiently low to prevent deposition on the tongue or in the back of the throat. Droplets below approximately 5-6 µm in diameter are transported almost completely by motion of the airstream and entrained air that carry them and not by their own momentum.

In certain aspects, the present disclosure includes and provides an ejector mechanism configured to eject a stream of droplets within the respirable range of less than about 5-6 µm, preferably less than about 5 µm. The ejector mechanism is comprised of an aperture plate configured to provide a desired surface contact angle. The aperture plate is directly or indirectly coupled to a piezoelectric actuator. In certain implementations, the aperture plate may be coupled to an actuator plate that is coupled to the piezoelectric actuator. The aperture plate generally includes a plurality of openings formed through its thickness and the piezoelectric actuator directly or indirectly (e.g. via an actuator plate) oscillates the aperture plate, having fluid in contact with one surface of the aperture plate, at a frequency and voltage to generate a directed aerosol stream of droplets through the openings of the aperture plate into the lungs, as the patient inhales. In other implementations where the aperture plate is coupled to the actuator plate, the actuator plate is oscillated by the piezoelectric oscillator at a frequency and voltage to generate a directed aerosol stream or plume of aerosol droplets.

In certain aspects, the present disclosure relates to a droplet delivery device for delivering a fluid as an ejected stream of droplets to the pulmonary system of a subject. In certain aspects, the therapeutic agents may be delivered at a high dose concentration and efficacy, as compared to alternative dosing routes and standard inhalation technologies.

In certain aspects, the droplet delivery device is capable of delivering a defined volume of fluid in the form of an ejected stream of droplets such that an adequate and repeatable high percentage of the droplets are delivered into the desired location within the airways, e.g., the alveolar airways of the subject during use.

As discussed above, effective delivery of droplets deep into the lung airways require droplets that are less than about 5-6 microns in diameter, specifically droplets with mass mean aerodynamic diameters (MMAD) that are less than about 5 microns. The mass mean aerodynamic diameter is defined as the diameter at which 50% of the droplets by mass are larger and 50% are smaller. In certain aspects of the disclosure, in order to deposit in the alveolar airways, droplets in this size range must have momentum that is sufficiently high to permit ejection out of the device, but sufficiently low to overcome deposition onto the tongue (soft palate) or pharynx.

In other aspects of the disclosure, methods for generating an ejected stream of droplets for delivery to the pulmonary system of user using the droplet delivery devices of the disclosure are provided. In certain embodiments, the ejected stream of droplets is generated in a controllable and defined droplet size range. By way of example, the droplet size range includes at least about 50%, at least about 60%, at least about 70%, at least about 85%, at least about 90%, between about 50% and about 90%, between about 60% and about 90%, between about 70% and about 90%, etc., of the ejected droplets are in the respirable range of below about 5 µm.

In other embodiments, the ejected stream of droplets may have one or more diameters, such that droplets having multiple diameters are generated so as to target multiple regions in the airways (mouth, tongue, throat, upper airways, lower airways, deep lung, etc.) By way of example, droplet diameters may range from about 1 µm to about 200 µm, about 2 µm to about 100 µm, about 2 µm to about 60 µm, about 2 µm to about 40 µm, about 2 µm to about 20 µm, about 1 µm to about 5µm, about 1 µm to about 4.7 µm, about 1 µm to about 4 µm, about 10 µm to about 40 µm, about 10 µm to about 20 µm, about 5 µm to about 10 µm, and combinations thereof. In particular embodiments, at least a fraction of the droplets have diameters in the respirable range, while other droplets may have diameters in other sizes so as to target non-respirable locations (e.g., larger than 5 µm). Illustrative ejected droplet streams in this regard might have 50% - 70% of droplets in the respirable range (less than about 5 µm), and 30% -50% outside of the respirable range (about 5 µm - about 10 µm, about 5 µm - about 20 µm, etc.)

In certain aspects of the disclosure, a droplet delivery device for delivery an ejected stream of droplets to the pulmonary system of a subject is provided. The droplet delivery device generally includes a housing and a reservoir disposed in or in fluid communication with the housing, an ejector mechanism in fluid communication with the reservoir, and at least one differential pressure sensor positioned within the housing. The differential pressure sensor is configured to electronically breath activate the ejector mechanism upon sensing a pre-determined pressure change within the housing, and the ejector mechanism is configured to generate a controllable plume of an ejected stream of droplets. The ejected stream of droplets is formed from low surface tension compositions, particularly compositions comprising agents that are insoluble or sparingly soluble in water. The ejector mechanism comprises a piezoelectric actuator which is directly or indirectly coupled to an aperture plate configured to provide a desired surface contact angle of greater than 80 degrees and having a plurality of openings formed through its thickness. The piezoelectric actuator is operable to directly or indirectly oscillate the aperture plate at a frequency to thereby generate an ejected stream of droplets.

In certain embodiments, the droplet delivery device may be configured in an in-line orientation in that the housing, ejector mechanism and related electronic components are orientated in a generally in-line or parallel configuration so as to form a small, hand-held device.

In certain embodiments, the droplet delivery device may include a combination reservoir/ejector mechanism module that may be replaceable or disposable either on a periodic basis, e.g., a daily, weekly, monthly, as-needed, etc. basis, as may be suitable for a prescription or over-the-counter medication.

The present disclosure also provides a droplet delivery device that is altitude insensitive. In certain implementations, the droplet delivery device is configured so as to be insensitive to pressure differentials that may occur when the user travels from sea level to sub-sea levels and at high altitudes, e.g., while traveling in an airplane where pressure differentials may be as great as 4 psi. As will be discussed in further detail herein, in certain implementations of the disclosure, the droplet delivery device may include a superhydrophobic filter, optionally in combination with a spiral vapor barrier, which provides for free exchange of air into and out of the reservoir, while blocking moisture or fluids from passing into the reservoir, thereby reducing or preventing fluid leakage or deposition on aperture plate surfaces.

In certain embodiments, the droplet delivery device is comprised of combination fluid reservoir/ejector mechanism, and a handheld unit containing a differential pressure sensor, a microprocessor and three AAA batteries. The microprocessor controls dose delivery, dose counting and software designed monitoring parameters that can be transmitted through wireless communication technology. The ejector mechanism may optimize droplet delivery to the user by creating droplets in a predefined droplet size range with a high degree of accuracy and repeatability.

In certain aspects, the droplet delivery device further includes a surface tension plate between the aperture plate and the reservoir, wherein the surface tension plate is configured to increase contact between the volume of fluid and the aperture plate. In other aspects, the ejector mechanism and the surface tension plate are configured in parallel orientation. In yet other aspects, the surface tension plate is located within 2 mm of the aperture plate so as to create sufficient hydrostatic force to provide capillary flow between the surface tension plate and the aperture plate.

In certain aspects, the devices of the disclosure eliminate the need for patient / device coordination by using a differential pressure sensor to initiate the piezoelectric ejector in response to the onset of inhalation. The device does not require manual triggering of droplet delivery. Further, droplet delivery from the devices of the disclosure are generated having little to no intrinsic velocity from the aerosol formation process and are inspired into the lungs solely by the user's incoming breath passing through the mouthpiece tube. The droplets ride on entrained air, providing improved deposition in the lung.

In certain embodiments, as described in further detail herein, when the fluid reservoir is mated to the handheld body, electrical contact is made between the base containing the batteries and the ejector mechanism. In certain embodiments, visual and audio indicators (e.g., LEDs and a small speaker) within the handheld base provide user notifications. By way of non-limiting example, the device may be, e.g., 3.5 cm high, 5 cm wide, 10.5 cm long and may weight approximately 95 grams with an empty fluid reservoir and with batteries inserted.

As described herein, in certain embodiments, an easily accessible on/off slide bar or power push button may activate the device and unseals the ejector mechanism. Visual indicators may indicate power and the number of remaining doses may be shown on an optional dose counter numerical display, indicating the unit is energized and ready to be used.

As the user inhales through the unit, the differential pressure sensor detects flow, e.g., by measuring the pressure drop across a Venturi plate at the back of the mouthpiece. When a desired pressure decline (8 liters/minute) is attained, the microprocessor activates the ejector mechanism, at which point visual and/or audio indicators may alert the user that dosing has started. The microprocessor may stop the ejector mechanism, e.g., 1.45 seconds after initiation (or at a designated time so as to achieve a desired administration dosage). In certain embodiments, as described in further detail herein, the device may then emit a positive chime sound after the initiation of dosing, indicating to the user to begin holding their breath for a designated period of time, e.g., 10 seconds. During the breath hold period, other visual or audio indicators may be presented to the user, e.g., the three green LEDs may blink. Additionally, there may be voice commands instructing the user as to proper times to exhale, inhale and hold their breath.

In certain embodiments, the slide switch or power button may also open (power on) / closes (power off) a sliding door on the handheld unit that seals the ejector mechanism for added security and sterility. In the closed (off) state, the ejector mechanism may be sealed from airborne contamination and potential evaporative effects. In certain embodiments, optional voice command and Instructions for Use may direct the user to slide the switch or power button to the off position (door closed) at the end of use. If the unit has not been turned off, after a time out period, e.g., 20 seconds of inactivity, the user may be reminded to slide the door closed or power off by lights and sounds.

Several features of the device allow precise dosing of specific droplet sizes. Droplet size is set by the diameter of the openings in the aperture plate which are formed with high accuracy. By way of example, the openings in the aperture plate may range in size from 1 µm to 6 µm, from 2 µm to 5 µm, from 3 µm to 5 µm, from 3 µm to 4 µm, etc. In certain embodiments, the aperture plate may include openings having different cross-sectional shapes or diameters to thereby provide ejected droplets having different average ejected droplet diameters.

By way of example, droplet diameters (and thereby opening diameter) may range from about 1 µm to about 200 µm, about 2 µm to about 100 µm, about 2 µm to about 60 µm, about 2 µm to about 40 µm, about 2 µm to about 20 µm, about 1 µm to about 5µm, about 1 µm to about 4.7 µm, about 1 µm to about 4 µm, about 10 µm to about 40 µm, about 10 µm to about 20 µm, about 5 µm to about 10 µm, and combinations thereof. In particular embodiments, at least a fraction of the droplets have diameters in the respirable range, while other droplets may have diameters in other sizes so as to target non-respirable locations (e.g., larger than about 5 µm). Illustrative ejected droplet streams in this regard might have 50% - 70% of droplets in the respirable range (less than about 5 µm), and 30% -50% outside of the respirable range (about 5 µm - about 10 µm, about 5 µm - about 20 µm, etc.)

Ejection rate, in droplets per second, is fixed by the frequency of the plate vibration, e.g., 108-kHz, which is actuated by the microprocessor. In certain embodiments, there is less than a 50-millisecond lag between the detection of the start of inhalation and full droplet generation.

Droplet production within the respirable range occurs early in the inhalation cycle, thereby minimizing the amount of droplets being deposited in the mouth or upper airways at the end of an inhalation. The design of the droplet delivery device maintains constant solution contact with the ejector mechanism, thus obviating the need for shaking and priming. Further, the ejector mechanism configuration (including the hydrophobic coating at the exit side surface) and the vent configuration on the fluid reservoir limit solution evaporation.

The microprocessor in the device ensures exact timing and actuation of the piezoelectric and records the date-time of each ejection event as well as the user's inhalation flow rate during the dose inhalation. A numerical display on the handheld base unit will indicate the number of doses remaining in the drug cartridge. The base unit will sense when a new cartridge has been inserted based on the unique electrical resistance of each individual cartridge. Dose counting and lockouts will be preprogramed into the microprocessor.

The device is constructed with materials currently used in FDA cleared devices. Manufacturing methods are employed to minimize extractables.

By way of example, the aperture plate can formed of a metal, e.g., stainless steel, nickel, cobalt, titanium, iridium, platinum, or palladium or alloys thereof. Alternatively, the plate can be formed of suitable polymeric material, and be coated or treated as noted above to achieve the desired contact angle, e.g., More particularly, the aperture plate may be composed of a material selected from the group consisting of poly ether ether ketone (PEEK), polyimide, polyetherimide, polyvinylidine fluoride (PVDF), ultra-high molecular weight polyethylene (UHMWPE), nickel, nickel-cobalt, nickel-palladium, palladium, platinum, metal alloys thereof, and combinations thereof. Further, in certain aspects, the aperture plate may comprise a domed shape.

The fluid reservoir is constructed of any suitable materials for the intended medical use. In particular, the fluid contacting portions are made from material compatible with the desired agent(s). By way of example, in certain embodiments, the agents only contact the inner side of the fluid reservoir and the inner face of the aperture plate and piezo drive. Wires connecting the piezoelectric ejector to the batteries contained in the base unit are embedded in the fluid reservoir shell to avoid contact with the agents. The piezoelectric ejector is attached to the fluid reservoir by a flexible bushing. The bushing contacts the agent and may be, e.g., any suitable material known in the art for such purposes such as those used in piezoelectric nebulizers.

The device mouthpiece, may be removable, replaceable and may be cleaned. Similarly, the device housing and fluid reservoir can be cleaned by wiping with a moist cloth. The ejector plate is recessed into the ampoule and cannot be damaged without removing the ampoule from the base and directly striking the sprayer with a sharp object.

Any suitable material may be used to form the housing of the droplet delivery device. In particular embodiment, the material should be selected such that it does not interact with the components of the device or the fluid to be ejected. For example, polymeric materials suitable for use in pharmaceutical applications may be used including, e.g., gamma radiation compatible polymer materials such as polystyrene, polysulfone, polyurethane, phenolics, polycarbonate, polyimides, aromatic polyesters (PET, PETG), etc.

In certain aspects of the disclosure, an electrostatic coating may be applied to the one or more portions of the housing, e.g., inner surfaces of the housing along the airflow pathway, to aid in reducing deposition of ejected droplets during use due to electrostatic charge build-up. Alternatively, one or more portions of the housing may be formed from a charge-dissipative polymer. For instance, conductive fillers are commercially available and may be compounded into the more common polymers used in medical applications, for example, PEEK, polycarbonate, polyolefins (polypropylene or polyethylene), or styrenes such as polystyrene or acrylic-butadiene-styrene (ABS) copolymers.

As described in further detail herein, the droplet delivery device of the disclosure may detect inspiratory airflow and record/store inspiratory airflow in a memory (on the device, smartphone, App, computer, etc.). A preset threshold (e.g., 8 L/min) triggers delivery of medication over a defined period of time, e.g., 1.5 seconds. Inspiratory flow is sampled frequently until flow stops. The number of times that delivery is triggered is incorporated and displayed in the dose counter LED on the device. Blue tooth capabilities permit the wireless transmission of the data.

Wireless communication (e.g., Bluetooth, wifi, cellular, etc.) in the device may communicate date, time and number of actuations per session to the user's smartphone. Software programing can provide charts, graphics, medication reminders and warnings to patients and whoever is granted permission to the data. The software application will be able to incorporate multiple agents that use the device of the disclosure.

The device of the present disclosure is configured to dispense droplets during the correct part of the inhalation cycle, and can including instruction and/or coaching features to assist patients with proper device use, e.g., by instructing the holding of breath for the correct amount of time after inhalation. The device of the disclosure allows this dual functionality because it both monitors air flow during the inhalation, and has internal sensors/controls which detect the end of inhalation (based upon measured flow rate) and can cue the patient to hold their breath for a fixed duration after the inhalation ceases.

In one exemplary embodiment, a patient may be coached to hold their breath with an LED that is turned on at the end of inhalation and turned off after a defined period of time (i.e., desired time period of breath hold), e.g., 10 seconds. Alternatively, the LED may blink after inhalation, and continue blinking until the breath holding period has ended. In this case, the processing in the device detects the end of inhalation, turns on the LED (or causes blinking of the LED, etc.), waits the defined period of time, and then turns off the LED. Similarly, the device can emit audio indications, e.g., one or more bursts of sound (e.g., a 50 millisecond pulse of 1000 Hz), verbal instructions to hold breath, verbal countdown, music, tune, melody, etc., at the end of inhalation to cue a patient to hold their breath for the during of the sound signals. If desired, the device may also vibrate during or upon conclusion of the breath holding period.

Ideally, the device provides a combination of audio and visual methods (or sound, light and vibration) described above to communicate to the user when the breath holding period has begun and when it has ended. Or during the breath holding to show progress (e.g., a visual or audio countdown).

In other aspects, the device of the disclosure may provide coaching to inhale longer, more deeply, etc. The average peak inspiratory flow during inhalation (or dosing) can be utilized to provide coaching. For example, a patient may hear a breath deeper command until they reach 90% of their average peak inspiratory flow as measured during inspiration (dosing) as stored on the device, phone or in the cloud.

In addition, an image capture device, including cameras, scanners, or other sensors without limitation, e.g. charge coupled device (CCD), may be provided to detect and measure the ejected aerosol plume. These detectors, LED, delta P transducer, CCD device, all provide controlling signals to a microprocessor or controller in the device used for monitoring, sensing, measuring and controlling the ejection of a plume of droplets and reporting patient compliance, treatment times, dosage, and patient usage history, etc., via Bluetooth, for example.

In certain embodiments, the reservoir/cartridge module may include components that may carry information read by the housing electronics including key parameters such as ejector mechanism functionality, drug identification, and information pertaining to patient dosing intervals. Some information may be added to the module at the factory, and some may be added at the pharmacy. In certain embodiments, information placed by the factory may be protected from modification by the pharmacy. The module information may be carried as a printed barcode or physical barcode encoded into the module geometry (such as light transmitting holes on a flange which are read by sensors on the housing). Information may also be carried by a programmable or non-programmable microchip on the module which communicates to the electronics in the housing.

By way of example, module programming at the factory or pharmacy may include a drug code which may be read by the device, communicated via wireless communication to an associated user smartphone and then verified as correct for the user. In the event a user inserts an incorrect, generic, damaged, etc., module into the device, the smartphone might be prompted to lock out operation of the device, thus providing a measure of user safety and security not possible with passive inhaler devices. In other embodiments, the device electronics can restrict use to a limited time period (perhaps a day, or weeks or months) to avoid issues related to drug aging or build-up of contamination or particulates within the device housing.

The droplet delivery device may further include various sensors and detectors to facilitate device activation, spray verification, patient compliance, diagnostic mechanisms, or as part of a larger network for data storage, big data analytics and for interacting and interconnected devices used for subject care and treatment, as described further herein. Further, the housing may include an LED assembly on a surface thereof to indicate various status notifications, e.g., ON/READY, ERROR, etc.

Reference will now be made to the figures, with like components illustrated with like references numbers.

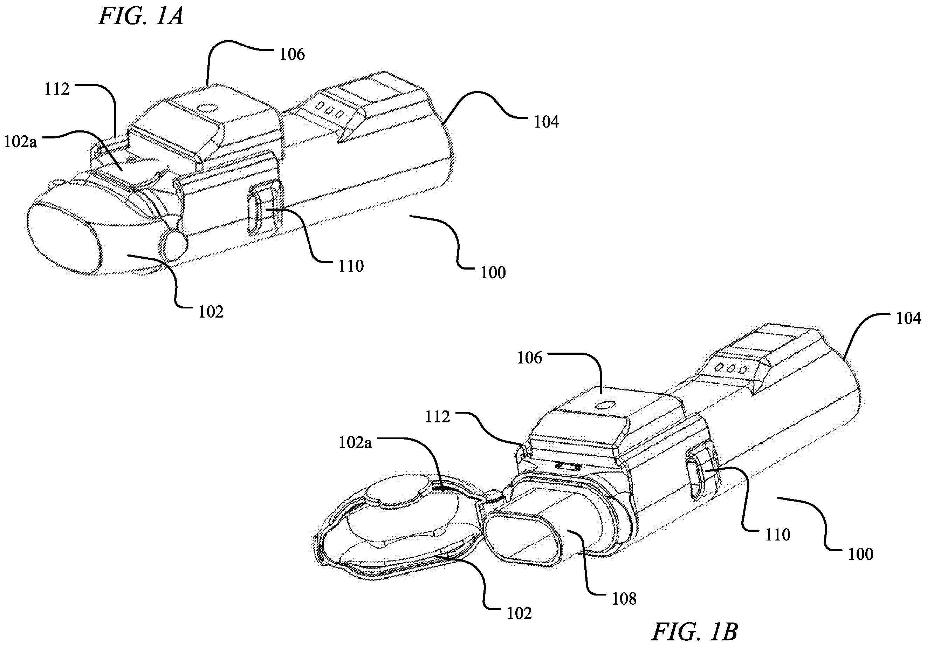

**FIGS. 1A and 1B** illustrate an exemplary droplet delivery device of the disclosure, with **FIG. 1A** showing the droplet delivery device 100 having a mouthpiece cover 102 in the closed position, and **FIG. 1B** having a mouthpiece cover 102 in the open position. As shown, the droplet delivery device is configured in an in-line orientation in that the housing, its internal components, and various device components (e.g., the mouthpiece, air inlet flow element, etc.) are orientated in a substantially in-line or parallel configuration (e.g., along the airflow path) so as to form a small, hand-held device.

In the embodiment shown in **FIGS. 1A** and **1B****,** the droplet delivery device 100 includes a base unit 104 and a fluid reservoir/ejector mechanism module 106. As illustrated in this embodiment, and discussed in further detail herein, the fluid reservoir 106 slides into the front of the base unit 104 via slides 112. In certain embodiments, mouthpiece cover 102 may include a push element 102a that facilitates insertion of fluid reservoir 106. Also illustrated are one or more airflow entrances or openings 110. By way of example, there may be airflow entrances on the opposite side of the device, multiple airflow entrances on the same side of the device, or a combination thereof (not shown). The droplet delivery device 100 also includes mouthpiece 108 at the airflow exit side of the device.

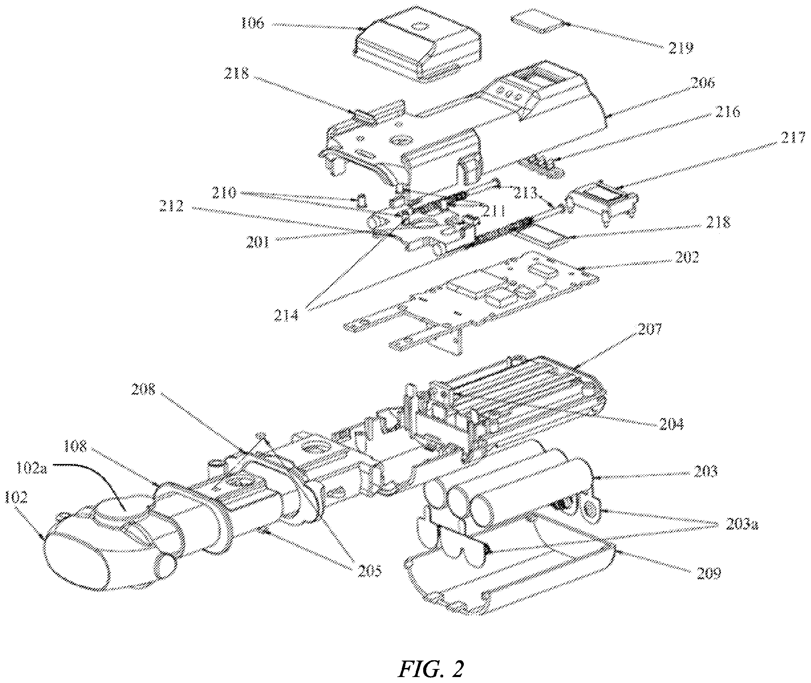

With reference to **FIG. 2****,** an exploded view of the exemplary droplet delivery device of **FIGS. 1A** and **1B** is shown, including internal components of the housing including a power/activation button 201; an electronics circuit board 202; a fluid reservoir/ejector mechanism module 106 that comprises an ejector mechanism (not shown) and reservoir; and a power source 203 (e.g., three AAA batteries, which may optionally be rechargeable) along with associated contacts 203a. In certain embodiments, the reservoir may be single-unit dose or multi-unit dose that may be replaceable, disposable or reusable. Also shown, one or more pressure sensors 204 and optional spray sensors 205. In certain embodiments, the device may also include various electrical contacts 210 and 211 to facilitate activation of the device upon insertion of drug delivery ampoule 106 into the base unit. Likewise, in certain embodiments, the device may include slides 212, posts 213, springs 214, and ampoule lock 215 to facilitate insertion of drug delivery ampoule 106 into the base unit.

The components may be packaged in a housing, and generally oriented in an in-line configuration. The housing may be disposable or reusable, single-dose or multi-dose. Although various configurations to form the housing are within the scope of the disclosure, as illustrated in **FIG. 2****,** the housing may comprise a top cover 206, a bottom cover 207, and an inner housing 208. The housing may also include a power source housing or cover 209.

In certain embodiments, the device may include audio and/or visual indications, e.g., to provide instructions and communications to a user. In such embodiments, the device may include a speaker or audio chip (not shown), one or more LED lights 216, and LCD display 217 (interfaced with an LCD control board 218 and lens cover 219). The housing may be handheld and may be adapted for communication with other devices via a Bluetooth communication module or similar wireless communication module, e.g., for communication with a subject's smart phone, tablet or smart device (not shown).

In certain embodiments, an air inlet flow element (not shown) may be positioned in the airflow at the airflow entrance of the housing and configured to facilitate non-turbulent (i.e., laminar and/or transitional) airflow across the exit side of aperture plate and to provide sufficient airflow to ensure that the ejected stream of droplets flows through the droplet delivery device during use. The air inlet flow element may comprise one or more openings formed there through and may be configured to increase or decrease internal pressure resistance within the droplet delivery device during use. In come embodiments, the air inlet flow element may comprises an array of one or more openings. In other embodiments, the air inlet flow element may comprise one or more baffles. In certain aspects, the one or more baffles may comprise one or more airflow openings.

In some embodiments, the air inlet flow element may be positioned within the mouthpiece. In other embodiments, the air inlet flow element may be positioned behind the exit side of the aperture plate along the direction of airflow. In yet other embodiments, the air inlet flow element is positioned in-line or in front of the exit side of the aperture plate along the direction of airflow.

Aspects of the present embodiment further allows customizing the internal pressure resistance of the particle delivery device by allowing the placement of laminar flow elements having openings of different sizes and varying configurations to selectively increase or decrease internal pressure resistance, as will be explained in further detail herein.

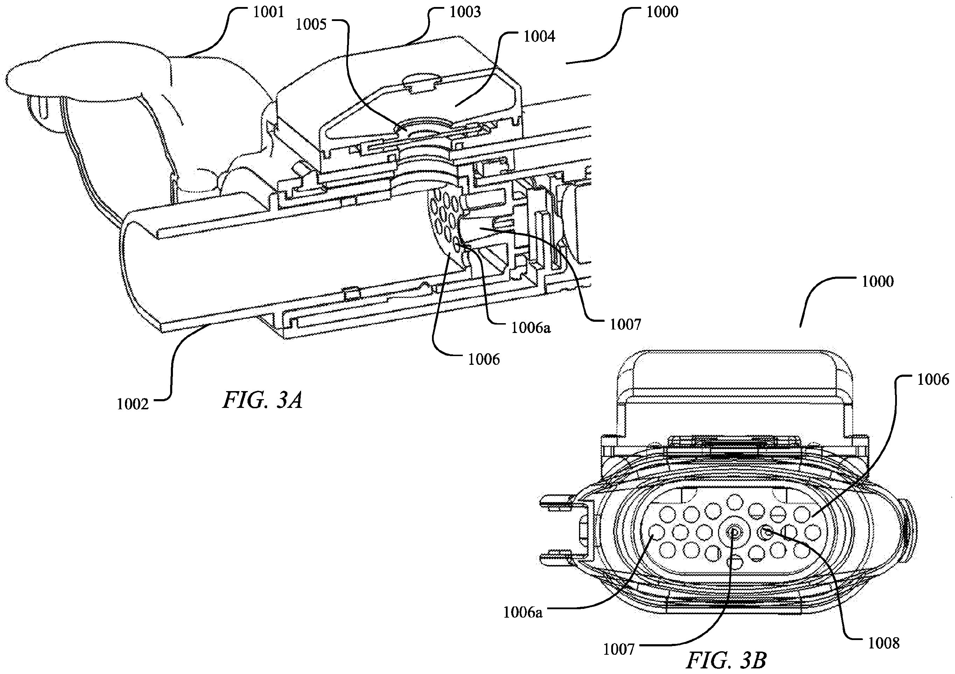

**FIGS. 3A-3C** illustrate certain exemplary air inlet flow elements of the disclosure. **FIGS. 3A-3C** also illustrate the position of pressure sensors, the mouthpiece, and air channels for reference pressure sensing. However, the disclosure is not so limited, and other configurations including those described herein are contemplated as within the scope of the disclosure. While not being so limited, the air inlet flow elements of **FIGS. 3A-3C** are particularly suitable for use with the droplet delivery devices of **FIGS. 1A-1B****.**

More particularly, **FIG. 3A** illustrates a cross-section of a partial in-line droplet delivery device 1000 of the disclosure including a mouthpiece cover 1001, a mouthpiece 1002, a drug delivery ampoule 1003 comprising a drug reservoir 1004 and an ejector mechanism 1005. As illustrated, the droplet delivery device includes an air inlet flow element 1006 having an array of holes 1006a at the air entrance of the mouthpiece 1002. Also shown is a pressure sensor port 1007, which may be used to sense a change in pressure within the mouthpiece. With reference to **FIG. 3B****,** a front view of the device 1000 is illustrated, wherein a second pressure sensor port 1008 is shown to provide for sensing of a reference or ambient pressure.

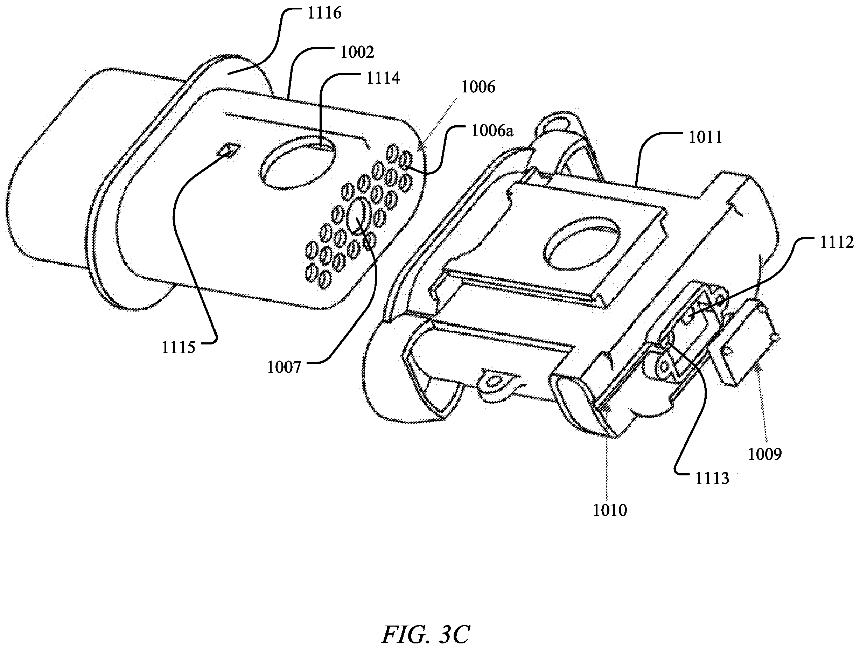

**FIG. 3C** illustrates a partial exploded view including mouthpiece 1002 and inner housing 1011. As shown, mouthpiece 1002 includes air intake flow element 1006 with an array of holes 1006a, and pressure sensor port 1007. Further, mouthpiece 1002 may include an ejection port 1114 positioned, e.g., on the top surface of the mouthpiece so as to align with the ejector mechanism to allow for ejection of the stream of droplets into the airflow of the device during use. Other sensor ports 1115 may be positioned as desired along the mouthpiece to allow for desired sensor function, e.g., spray detection. The mouthpiece may also include positioning baffle 1116 that interfaces with the base unit upon insertion. Inner housing 1011 includes pressure sensor board 1009 and outside channel 1010 for facilitating sensing of reference or ambient pressure. The inner housing further includes a first pressure sensing port 1112 to facilitate sensing of pressure changes within the device (e.g., within the mouthpiece or housing), and a second pressure sensing port 1113 to facilitate sensing of reference or ambient pressure.

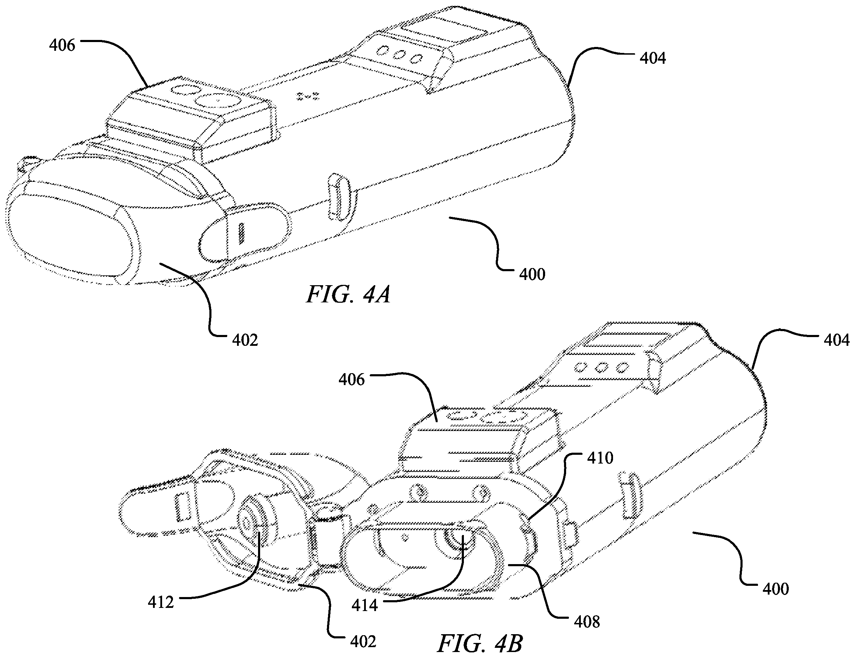

In another embodiment, **FIGS. 4A and 4B** illustrate an alternative droplet delivery device of the disclosure wherein the fluid reservoir/ejector mechanism module is inserted into the front of the base unit. With reference to **FIG. 4A** showing the droplet delivery device 400 with a base unit 404 having a mouthpiece cover 402 in the closed position, and **FIG. 4B** with a base unit 404 having a mouthpiece cover 402 in the open position. As shown, the droplet delivery device is configured in an in-line orientation in that the housing, its internal components, and various device components (e.g., the mouthpiece, air inlet flow element, etc.) are orientated in a substantially in-line or parallel configuration (e.g., along the airflow path) so as to form a small, hand-held device.

In the embodiment shown in **FIGS. 4A** and **4B****,** the droplet delivery device 400 includes a base unit 404 and a fluid reservoir 406. As illustrated in this embodiment, and discussed in further detail herein, the fluid reservoir 406 slides into the front of the base unit 404. In certain embodiments, mouthpiece cover 402 may include aperture plate plug 412 which may cover aperture plate 414 when cover 402 is in a closed position. Also illustrated are one or more airflow entrances or openings 410 in mouthpiece 408. By way of example, there may be airflow entrances on the opposite side of the device, multiple airflow entrances on the same side of the device, or a combination thereof (not shown). The droplet delivery device 400 also includes mouthpiece 408 at the airflow exit side of the device.

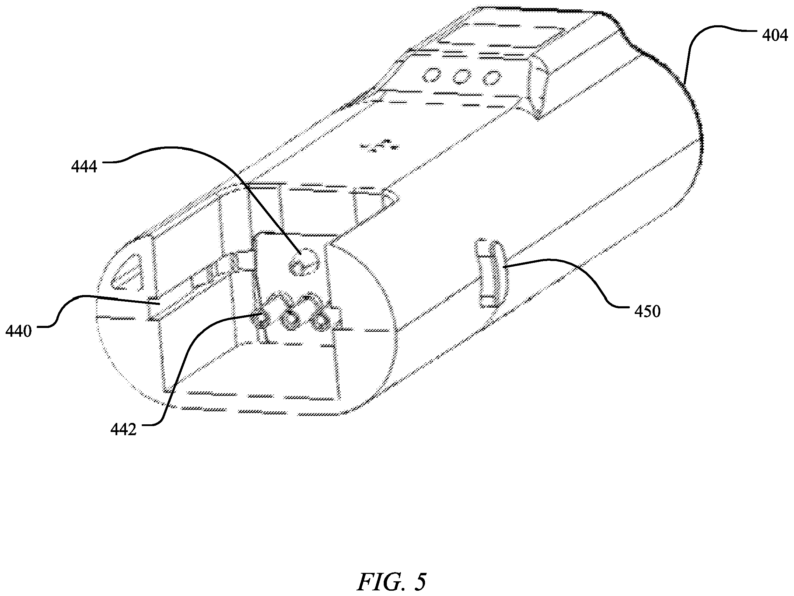

**FIG. 5** illustrates the base unit 404 of the embodiment of **FIGS. 4A** and **4B** without the fluid reservoir/ejector mechanism module inserted. Without the fluid reservoir/ejector mechanism module inserted, tracks 440 for directing the module into place, electrical contacts 442, and sensor port 444 are shown. Also shown is release button 450.

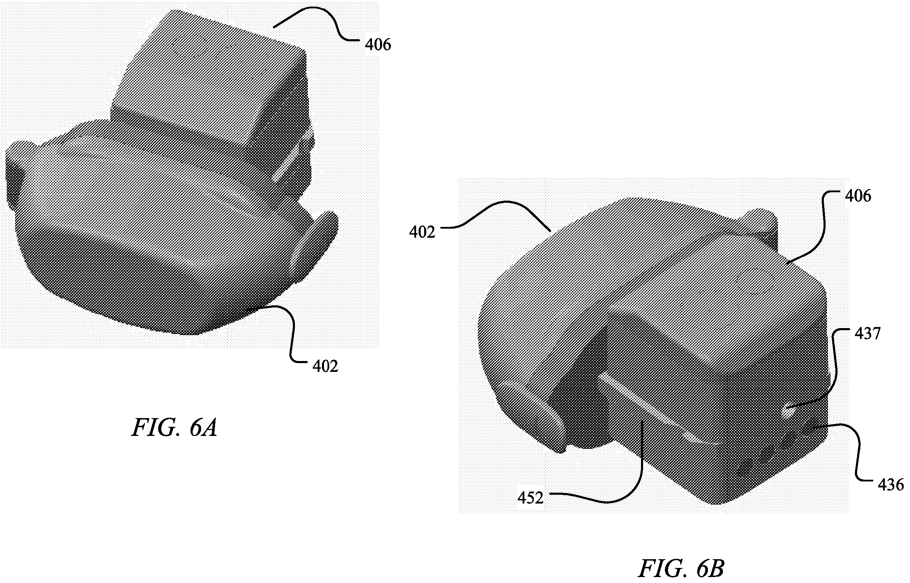

**FIGS. 6A and 6B** illustrate a fluid reservoir/ejector mechanism module 406 with mouthpiece cover 402 attached and in a closed position in front view (**FIG. 6A**) and back view (**FIG. 6B**). **FIG. 6B** illustrates electrical contacts 436 and sensor port 437 of the module, as well as protruding slides 452 to facilitate placement of the module into tracks 440 during insertion. By way of example, when fluid reservoir/ejector mechanism module 406 is inserted into base unit 404, protruding slides 452 mate with tracks 440, sensor port 437 mates with sensor port 444, and electrical contacts 436 mates with electrical contacts 442. The fluid reservoir/ejector mechanism module is pushed into the base unit and locked into place with the protruding slides and tracks engaging one another. During use, a pressure sensor located on the control board senses pressure changes within the device via the pressure sensing ports (e.g., within the mouthpiece). To facilitate detection of pressure changes, the base unit includes a second pressure sensing port and outside channel (not shown) to facilitate sensing of reference or ambient pressure.

Again, in certain embodiments, an air inlet flow element (not shown) may be positioned in the airflow at the airflow entrance of the housing and configured to facilitate non-turbulent (i.e., laminar and/or transitional) airflow across the exit side of aperture plate and to provide sufficient airflow to ensure that the ejected stream of droplets flows through the droplet delivery device during use. The air inlet flow element may comprise one or more openings formed there through and may be configured to increase or decrease internal pressure resistance within the droplet delivery device during use. In come embodiments, the air inlet flow element may comprises an array of one or more openings. In other embodiments, the air inlet flow element may comprise one or more baffles. In certain aspects, the one or more baffles may comprise one or more airflow openings.

In some embodiments, the air inlet flow element may be positioned within the mouthpiece. In other embodiments, the air inlet flow element may be positioned behind the exit side of the aperture plate along the direction of airflow. In yet other embodiments, the air inlet flow element is positioned in-line or in front of the exit side of the aperture plate along the direction of airflow.

Aspects of the present embodiment further allows customizing the internal pressure resistance of the particle delivery device by allowing the placement of laminar flow elements having openings of different sizes and varying configurations to selectively increase or decrease internal pressure resistance.

As illustrated in the various embodiments of the figures, in certain embodiments of the droplet device, the housing and ejector mechanism are oriented such that the exit side of the aperture plate is perpendicular to the direction of airflow and the stream of droplets is ejected in parallel to the direction of airflow. In other embodiments, the housing and ejector mechanism are oriented such that the exit side of the aperture plate is parallel to the direction of airflow and the stream of droplets is ejected substantially perpendicularly to the direction of airflow such that the ejected stream of droplets is directed through the housing at an approximate 90 degree change of trajectory prior to expulsion from the housing.

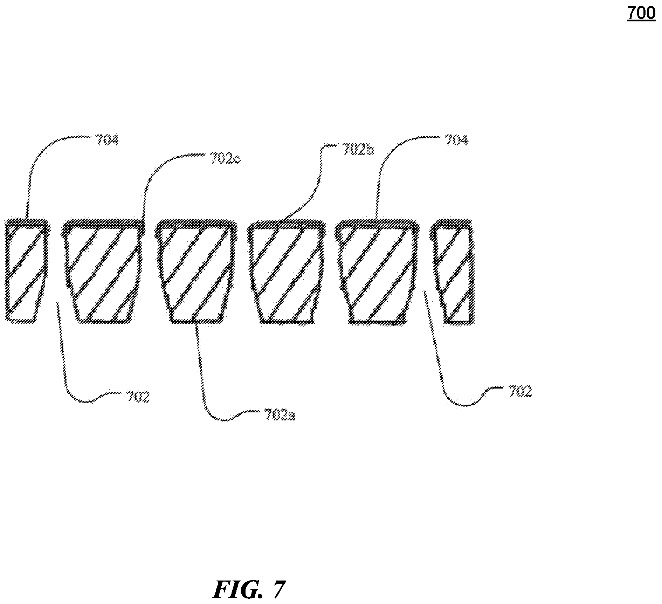

With reference to **FIG. 7****,** a cross-section of an opening 702 of an exemplary aperture plate 700 of the disclosure is illustrated. As shown, opening 702 is configured with a generally curved taper from droplet entrance 702a, to droplet exit 702b, and droplet entrance 702a is formed with a larger diameter/cross-sectional size than droplet exit 702b. However, the aperture plates of the disclosure are not limited to curved taper configurations, and any suitable cross-sectional shape or structure of the opening may be utilized in connection with the present disclosure.

Hydrophobic coating 704 is formed on droplet exit side of aperture plate 700. At least at a portion of the droplet exit surface, within at least one opening, at least at a portion of the droplet entrance surface, and combinations thereof. In particular embodiments, the aperture plate may provide the desired surface contact angle at least at a portion of the droplet exit surface or at least at a portion of the droplet exit surface and within at least one opening. In the embodiment illustrated, hydrophobic coating 704 is formed along the surface of droplet exit surface and along small portions 702c of the interior of openings 702 near the droplet exit area 702b.

While the devices of the disclosure are not so limited, the aperture plate may have a thickness of between about 100 µm and 300 µm, with openings having a maximum diameter at the droplet entrance side of 30 µm to 300 µm, and openings having a maximum diameter at the droplet exit side of about 0.5 µm to 6 µm (or more, depending on the desired droplet ejection diameter, as discussed herein). The thickness of the hydrophobic coating may be sufficient so as to achieve the desired surface contact angle, but not so thick so as to completely block the opening. By way of non-limiting example, the hydrophobic coating may range in thickness from about 50 nm to about 200 nm, about 50 nm to about 150 nm, about 75 nm to about 110 nm, etc. As will be recognized by those of skill in the art, the opening diameters may be adjusted to accommodate hydrophobic coating thickness, if desired.

### EXAMPLES

Ejector mechanisms with nickel-palladium alloy aperture plates were used to investigate the ability of aperture plates with controlled contact angles to eject low surface tension solutions. In general, nickel-palladium alloy exhibit contact angles of between about 40 and about 70 degrees. Aperture plates formed from such nickel-palladium alloys generate efficient aerosols from aqueous solutions in piezo driven droplet delivery devices of the disclosure. However, these aperture plates failed to generate aerosols of ethanol based solutions.

As such, in accordance with aspects of the disclosure, nickel-palladium alloy aperture plates were coated to modify surface contact angles between 89 degrees to evaluate ethanol droplet generation. Both sides of the aperture plates were coated with a process by PlasmaTreat gave an 89 degree contact angle. However, some of these aperture plates were found to leak.

In another embodiment, nickel-palladium alloy aperture plates were surface coating with a polytetrafluoroethylene (Teflon) coating that gave a 100 to 110 degree surface contact angle. Testing showed these aperture plates generate droplets at a mass flow rate of about 17 mG per second.

Testing was done with three aperture plates coated with polytetrafluoroethylene (Teflon) by Nordson March and three uncoated nickel-palladium aperture plates. The coated surfaces had a contact angle with water of 100 to 110 degrees. Tests were performed for mass ejection at 0%, 5%, 50%, 70%, and 100% ethanol in water (N=10 @ 28.3 slm). Mass ejection is measured by weighing the cartridge before and after dispense. Each ejector mechanism is dried after ten ejections to check for leakage through or around the aperture plate, and droplets that have deposited on surfaces of the ejector mechanism.

Results are presented in the table below as average ejection for ten, 1.5 second dispenses. Good ejection was found for both 100% and 5% ethanol, but no ejection with 50% or 70% ethanol-water solutions. Negligible leakage through the aperture plate was observed.

| | 1.5 second cartridge output (mG) | | |
|---|---|---|---|
| | C13 | C11 | C10 |
| 100% ethanol | 14.52 | 13.53 | 15.56 |
| 100% ethanol | 19.18 | 16.95 | 16.23 |
| 5% ethanol | 11.13 | 7.48 | 8.07 |
| 5% ethanol | 10.08 | 7.88 | 7.57 |

In other examples, devices with aperture plates coated with polytetrafluoroethylene (Teflon) and siloxane were tested. The coated surfaces had a contact angle with water of 100 to 130 degrees. Tests are performed for mass ejection at 5%, 10%, 30%, 50%, 70%, 90%, 95%, and 100% ethanol in water. Mass ejection is measured by weighing the cartridge before and after dispense. Each ejector mechanism is dried after ten ejections to check for leakage through or around the aperture plate, and droplets that have deposited on surfaces of the ejector mechanism.

Results are similar to those above, with good ejection for 100%, 95%, 90%, 10%, and 5%, but no ejection in the mid-range at 70%, 50%, and 30% ethanol in water.

The lack of ejection at the mid-range, i.e., 70%, 50% and 30% solutions, is consistent with the higher viscosity of these solutions. Additionally, no ejection was observed for any solutions for the uncoated nickel-palladium aperture plates.

The invention is defined in independent claim 1. Preferred embodiments are matter of the appended dependent claims.

## Claims

1. An electronically actuated droplet delivery device (100) for delivering a low surface tension composition as an ejected stream of droplets to the pulmonary system of a subject, the device comprising:
a housing;
a mouthpiece (108) positioned at an airflow exit of the device;
a reservoir (106) disposed within or in fluid communication with the housing containing said low surface tension composition;
an electronically actuated ejector mechanism in fluid communication with the reservoir (106) and configured to generate the ejected stream of droplets;
and at least one differential pressure sensor (204) positioned within the housing, the at least one differential pressure sensor (204) configured to activate the ejector mechanism upon sensing a pre-determined pressure change within the mouthpiece (108) to thereby generate the ejected stream of droplets;
the ejector mechanism comprising a piezoelectric actuator and an aperture plate (700), the aperture plate having and a plurality of openings (702) formed through its thickness, and the piezoelectric actuator operable to oscillate the aperture plate at a frequency to thereby generate the ejected stream of droplets;
wherein the ejector mechanism is configured to generate the ejected stream of droplets wherein at least about 50% of the droplets have an average ejected droplet diameter of less than about 6 microns, such that at least about 50% of the mass of the ejected stream of droplets is delivered in a respirable range to the pulmonary system of the subject during use;
**characterized in that** the low surface composition comprises an agent that is insoluble or sparingly soluble in water, and one or more surfaces of the aperture plate are configured to provide a surface contact angle of greater than 90 degrees.

2. The droplet delivery device (100) of claim 1, wherein the aperture plate (700) has one or more surfaces configured to provide a surface contact angle of between 90 degrees and 140 degrees.

3. The droplet delivery device (100) of claim 1 or 2, wherein the aperture plate (700) is coated with a hydrophobic polymer (704) to provide said surface contact angle.

4. The droplet delivery device (100) of claim 3, wherein the hydrophobic polymer (704) is selected from the group consisting of polytetrafluoroethylene, a siloxane, paraffin, and polyisobutylene.

5. The droplet delivery device (100) of claim 3 or 4, wherein the hydrophobic polymer (704) is coated on at least a portion of droplet exit side surface of the aperture plate or within at least a portion of the interior of at least one of the openings.

6. The droplet delivery device (100) of any of claims 1-5, wherein the low surface tension composition comprises an alcohol as a solvent.

7. The droplet delivery device (100) of any of claims 1-6, wherein the aperture plate (700) is composed of a material selected from the group consisting of poly ether ether ketone (PEEK), polyimide, polyetherimide, polyvinylidine fluoride (PVDF), ultra-high molecular weight polyethylene (UHMWPE), nickel, nickel-cobalt, nickel-palladium, palladium, platinum, metal alloys thereof, and combinations thereof.

8. The droplet delivery device (100) of any of claims 1-7, wherein one or more of the plurality of openings (702) have different cross-sectional shapes or diameters to thereby provide ejected droplets having different average ejected droplet diameters.

9. The droplet delivery device (100) of any of claims 1-8, wherein the agent that is insoluble or sparingly soluble in water is selected from the group consisting of cannabinoids and derivatives thereof, fluticasone furoate, and fluticasone propionate.

## Patentansprüche

1. Elektronisch betätigte Tröpfchenabgabevorrichtung (100) zum Abgeben einer Zusammensetzung mit geringer Oberflächenspannung als ausgestoßene Tröpfchenströmung an das Lungensystem eines Individuums, wobei die Vorrichtung Folgendes umfasst:
ein Gehäuse;
ein Mundstück (108), das an einem Luftströmungsausgang der Vorrichtung positioniert ist;
einen Behälter (106), der innerhalb des Gehäuses oder in Fluidkommunikation mit dem Gehäuse angeordnet ist und die Zusammensetzung mit geringer Oberflächenspannung enthält;
einen elektronisch betätigten Ausstoßmechanismus in Fluidkommunikation mit dem Behälter (106), der dazu ausgelegt ist, die ausgestoßene Tröpfchenströmung zu erzeugen; und
zumindest einen Differenzdrucksensor (204), der innerhalb des Gehäuses positioniert ist, wobei der zumindest eine Differenzdrucksensor (204) dazu ausgelegt ist, nach dem Abfühlen einer vorbestimmten Druckänderung innerhalb des Mundstücks (108) den Ausstoßmechanismus zu aktivieren, um so die ausgestoßene Tröpfchenströmung zu erzeugen;
wobei der Ausstoßmechanismus ein piezoelektrisches Betätigungselement und eine Aperturplatte (700) umfasst, wobei die Aperturplatte eine Vielzahl von Öffnungen (702), die durch ihre Dicke hindurch ausgebildet sind, aufweist und wobei das piezoelektrische Betätigungselement betreibbar ist, um die Aperturplatte mit einer Frequenz in Schwingung zu versetzen, um so die ausgestoßene Tröpfchenströmung zu erzeugen;
wobei der Ausstoßmechanismus dazu ausgelegt ist, die ausgestoßene Tröpfchenströmung zu erzeugen, wobei zumindest etwa 50 % der Tröpfchen einen mittleren Ausgestoßene-TröpfchenDurchmesser von weniger als etwa 6 Mikron aufweisen, so dass während des Gebrauchs zumindest etwa 50 % der Masse der ausgestoßenen Tröpfchenströmung in einem einatembaren Bereich an das Lungensystem des Individuums abgegeben werden;
**dadurch gekennzeichnet, dass** die Zusammensetzung mit geringer Oberflächenspannung ein Mittel umfasst, das in Wasser unlöslich oder schwerlöslich ist, und dass eine oder mehrere Oberflächen der Aperturplatte dazu ausgelegt sind, einen Oberflachenkontaktwinkel von größer als 90 Grad bereitzustellen.

2. Tröpfchenabgabevorrichtung (100) nach Anspruch 1, wobei die Aperturplatte (700) eine oder mehrere Oberflächen aufweist, die dazu ausgelegt sind, einen Oberflachenkontaktwinkel von zwischen 90 und 140 Grad bereitzustellen.

3. Tröpfchenabgabevorrichtung (100) nach Anspruch 1 oder 2, wobei die Aperturplatte (700) mit einem hydrophoben Polymer (704) beschichtet ist, um den Oberflachenkontaktwinkel bereitzustellen.

4. Tröpfchenabgabevorrichtung (100) nach Anspruch 3, wobei das hydrophobe Polymer (704) aus der Gruppe bestehend aus Polytetrafluorethylen, einem Siloxan, Paraffin und Polyisobutylen ausgewählt ist.

5. Tröpfchenabgabevorrichtung (100) nach Anspruch 3 oder 4, wobei das hydrophobe Polymer (704) auf zumindest einem Abschnitt einer Tröpfchenaustrittsseitenfläche der Aperturplatte oder innerhalb zumindest eines Abschnitts des Inneren von zumindest einer der Öffnungen aufgebracht ist.

6. Tröpfchenabgabevorrichtung (100) nach Anspruch 1 bis 5, wobei die Zusammensetzung mit geringer Oberflächenspannung einen Alkohol als Lösungsmittel umfasst.

7. Tröpfchenabgabevorrichtung (100) nach einem der Ansprüche 1 bis 6, wobei die Aperturplatte (700) aus einem Material besteht, das aus der Gruppe bestehend aus Polyetheretherketon (PEEK), Polyimid, Polyetherimid, Polyvinylidenfluorid (PVDF), ultrahochmolekularem Polyethylen (UHMWPE), Nickel, Nickel-Kobalt, Nickel-Palladium, Palladium, Platin, Metalllegierungen davon und Kombinationen davon ausgewählt ist.

8. Tröpfchenabgabevorrichtung (100) nach einem der Ansprüche 1 bis 7, wobei eine oder mehrere der Vielzahl von Öffnungen (702) verschiedene Querschnittsformen oder Durchmesser aufweisen, um so ausgestoßene Tröpfchen mit verschiedenen Ausgestoßene-Tröpfen-Durchmessern bereitzustellen.

9. Tröpfchenabgabevorrichtung (100) nach einem der Ansprüche 1 bis 8, wobei das Mittel, das in Wasser unlöslich oder schwerlöslich ist, aus der Gruppe bestehend aus Cannabinoiden und Derivaten davon, Fluticasonfuroat und Fluticasonpropionat ausgewählt ist.

## Revendications

1. Dispositif d'administration de gouttelettes actionné électroniquement (100) pour administrer une composition à faible tension de surface sous la forme d'un flux éjecté de gouttelettes dans le système pulmonaire d'un sujet, le dispositif comprenant :
un boîtier ;
un embout buccal (108) positionné au niveau d'une sortie d'écoulement d'air du dispositif ;
un réservoir (106) disposé à l'intérieur du boîtier, ou en communication fluidique avec celui-ci, contenant ladite composition à faible tension de surface ;
un mécanisme éjecteur actionné électroniquement en communication fluidique avec le réservoir (106) et configuré pour générer le flux éjecté de gouttelettes ; et
au moins un capteur de pression différentielle (204) positionné dans le boîtier, le au moins un capteur de pression différentielle (204) étant configuré pour activer le mécanisme éjecteur lors de la détection d'un changement de pression prédéterminé dans l'embout buccal (108) pour générer ainsi le flux éjecté de gouttelettes ;
le mécanisme éjecteur comprenant un actionneur piézoélectrique et une plaque d'ouvertures (700), la plaque d'ouvertures présentant une pluralité d'orifices (702) formées à travers son épaisseur, et l'actionneur piézoélectrique pouvant fonctionner pour faire osciller la plaque d'ouvertures à une fréquence pour générer ainsi le flux éjecté de gouttelettes ;
dans lequel le mécanisme éjecteur est configuré pour générer le flux éjecté de gouttelettes dans lequel au moins environ 50 % des gouttelettes présentent un diamètre moyen de gouttelettes éjectées inférieur à environ 6 microns, de telle sorte qu'au moins environ 50 % de la masse du flux éjecté de gouttelettes est administrée dans une plage respirable dans le système pulmonaire du sujet pendant l'utilisation ;
**caractérisé en ce que** la composition à faible tension de surface comprend un agent qui est insoluble ou modérément soluble dans l'eau, et une ou plusieurs surfaces de la plaque d'ouvertures sont configurées pour fournir un angle de contact de surface supérieur à 90 degrés.

2. Dispositif d'administration de gouttelettes (100) selon la revendication 1, dans lequel la plaque d'ouvertures (700) présente une ou plusieurs surfaces configurées pour fournir un angle de contact de surface compris entre 90 degrés et 140 degrés.

3. Dispositif d'administration de gouttelettes (100) selon la revendication 1 ou 2, dans lequel la plaque d'ouvertures (700) est revêtue d'un polymère hydrophobe (704) pour fournir ledit angle de contact de surface.

4. Dispositif d'administration de gouttelettes (100) selon la revendication 3, dans lequel le polymère hydrophobe (704) est sélectionné dans le groupe constitué de polytétrafluoroéthylène, d'un siloxane, de paraffine, et de polyisobutylène.

5. Dispositif d'administration de gouttelettes (100) selon la revendication 3 ou 4, dans lequel le polymère hydrophobe (704) est revêtu sur au moins une partie de la surface latérale de sortie de gouttelettes de la plaque d'ouvertures ou dans au moins une partie de l'intérieur d'au moins un des orifices.

6. Dispositif d'administration de gouttelettes (100) selon l'une quelconque des revendications 1 à 5, dans lequel la composition à faible tension de surface comprend un alcool en tant que solvant.

7. Dispositif d'administration de gouttelettes (100) selon l'une quelconque des revendications 1 à 6, dans lequel la plaque d'ouvertures (700) est composée d'un matériau choisi dans le groupe constitué de polyéther éther cétone (PEEK), de polyimide, de polyétherimide, de fluorure de polyvinylidine (PVDF), de polyéthylène de poids moléculaire ultra-élevé (UHMWPE), de nickel, de nickel-cobalt, de nickel-palladium, de palladium, de platine, d'alliages métalliques de ceux-ci, et de combinaisons de ceux-ci.

8. Dispositif d'administration de gouttelettes (100) selon l'une quelconque des revendications 1 à 7, dans lequel une ou plusieurs de la pluralité d'orifices (702) présentent des formes ou diamètres en coupe transversale différents pour fournir ainsi des gouttelettes éjectées présentant des diamètres moyens de gouttelettes éjectées différents.

9. Dispositif d'administration de gouttelettes (100) selon l'une quelconque des revendications 1 à 8, dans lequel l'agent qui est insoluble ou modérément soluble dans l'eau est choisi dans le groupe constitué de cannabinoïdes et dérivés de ceux-ci, de furoate de fluticasone, et de propionate de fluticasone.
